# EUROPÄISCHE PATENTSCHRIFT

(11) **EP 1 652 487 B1**
(45) Veröffentlichungstag und Bekanntmachung des Hinweises auf die Patenterteilung: **11.02.2009**
(21) Anmeldenummer: 04025401.3
(22) Anmeldetag: 26.10.2004
(51) Int. Cl.: A61B 19/00

(54) **Präkalibriertes Mehrweginstrument**
Pre-calibrated multi-function instrument
Instrument multifonctionnel pre-calibré

(43) Veröffentlichungstag der Anmeldung: 03.05.2006
(73) Patentinhaber: BrainLAB AG, 85622 Feldkirchen (DE)
(72) Erfinder: Neubauer, Timo, 85622 Feldkirchen (DE); Plassky, Norman, 99099 Erfurt (DE)
(74) Vertreter: Schwabe - Sandmair - Marx

(56) Entgegenhaltungen:
- WO-A-01/67979
- WO-A-03/061501
- WO-A-20/04016178
- FR-A- 2 830 743
- US-A1- 2004 068 179
- US-A1- 2004 158 260
- US-B1- 6 190 395

## Beschreibung

Die vorliegende Erfindung bezieht sich auf ein präkalibriertes Mehrweginstrument, insbesondere zum Einsatz im medizinischen Bereich, welches mehrere Instrumente aufweist, die insbesondere zum Registrieren von Knochen, zum Beispiel bei einem chirurgischen Eingriff, eingesetzt werden können. Marker sind auf dem Mehrweginstrument angeordnet, deren relative Lage zueinander von einem Navigationssystem erkannt werden kann, wobei aus der erkannten relativen Lage oder Anordnung der Marker auf das Instrument oder die Spitze bzw. das Ende des präkalibrierten Mehrweginstruments geschlossen werden kann, das verwendbar bzw. in einem einsetzbaren Zustand ist. Die vorliegende Erfindung betrifft des Weiteren ein Verfahren zum Erkennen des Zustandes eines präkalibrierten Mehrweginstrumentes, wobei die Lage der Marker zueinander erkannt wird und durch Vergleich der erkannten Markergeometrie mit vorher gespeicherten Markergeometrien darauf geschlossen werden kann, welches Instrument des präkalibrierten Mehrweginstrumentes eingesetzt werden kann und zum Beispiel aus einer Ummantelung herausragt.

Üblicherweise besitzen Instrumente, die einen Registrierungsvorgang ausführen können, eine feste Markeranordnung mit passiven oder aktiven Markern, wobei die Geometrie der Markeranordnung von einem Navigationssystem identifiziert werden kann, womit auf die Art des Instrumentes geschlossen werden kann. Für verschiedene Anwendungen existieren verschiedene Registrierungsinstrumente, wobei jedes Instrument einzeln kalibriert werden muss und jedes Instrument von dem System registriert oder erkannt werden muss.

Aus der US 6,190,395 B1 ist eine Bandklammer bekannt, welche mit einer Markeranordnung verbunden werden kann. In die Bandklammer kann ein Instrument eingeschoben werden.

Die WO 03/061501 A2 offenbart ein Verfahren und eine Vorrichtung zum Rekonstruieren von Knochenoberflächen während eines chirurgischen Eingriffes, wobei ein Instrument ein schmaleres und ein größeres Ende und eine Positions-Abtastungs-Vorrichtung aufweist.

Aus der FR-A-2830743 ist eine feststehende Anordnung aus drei Markem bekannt, wobei in Abhängigkeit von dem Druck auf eine Kontaktkugel einer der Marker mehr oder weniger weit aus einer Umhüllung herausgeschoben wird.

Es ist eine Aufgabe der vorliegenden Erfindung ein Instrument und ein Verfahren vorzuschlagen, welche eine Vereinfachung des Kalibriervorganges ermöglichen.

Diese Aufgabe wird durch die Gegenstände der unabhängigen Ansprüche gelöst. Vorteilhafte Ausführungsformen ergeben sich aus den Unteransprüchen.

Dabei wird unter Präkalibrierung im Sinne der Erfindung verstanden, dass die Geometrie des Mehrweginstrumentes bekannt ist, oder dass Daten bekannt sind, welche die Geometrie des Mehrweginstrumentes beschreiben, wobei die Daten insbesondere einem Navigationssystem oder der Software eines Navigationssystems bekannt sind und zum Beispiel in einem Speicher gespeichert oder zwischengespeichert werden können.

Diese Daten können zum Beispiel Positions- oder Längenangaben sein, die zum Beispiel die Spitzen oder Enden oder Funktionselemente des Mehrweginstrumentes kennzeichnen, oder angeben, wie lang das Instrument ist oder an welcher Position des Mehrweginstrumentes sich die Spitzen oder Enden oder die Funktionselemente befinden oder können insbesondere angegeben wie die Spitzen oder Enden oder Funktionselemente des Instrumentes geformt sind.

Die Daten, welche die Geometrie des Mehrweginstrumentes beschreiben, können auch Informationen über die Position oder die Lage der Marker auf dem Mehrweginstrument enthalten und können enthalten, welche Markeranordnung oder Markergeometrie mit welchem Funktionselement korrespondiert oder können die Einsatzmöglichkeit eines Funktionselementes angeben. Folglich könnte zum Beispiel ein Navigationssystem eine Markergeometrie erfassen, woraus direkt geschlossen werden kann, welches Funktionselement eingesetzt werden kann oder soll, oder welche Form das einsetzbare Funktionselement hat.

Das erfindungsgemäße präkalibrierte Mehrweginstrument umfasst einen Instrumentenhalter an oder in dem ein Instrument angeordnet ist, welches relativ zu dem Instrumentenhalter bewegt werden kann und mindestens drei Marker, welche sowohl aktiv als auch passiv sein können, wobei mindestens einer der Marker zusammen mit dem Instrument relativ zu dem Instrumentenhalter bewegt werden kann. Dabei kann mindestens einer der Marker zum Beispiel mit dem Instrument verbunden sein oder direkt auf diesem angeordnet sein, so dass der Marker vorzugsweise um einen Vektor gleicher Richtung und gleichen Betrags bewegt wird wie das Instrument oder die Strecken, um die der Marker und das Instrument verschoben werden, in Richtung und Länge übereinstimmen. Mindestens zwei der Marker können mit dem Instrumentenhalter verbunden sein oder insbesondere fest oder unbeweglich direkt auf dem Instrumentenhalter angeordnet sein, so dass das Instrument und der mit dem Instrument verbundene Marker eine Relativbewegung zu dem Instrumentenhalter und den mindestens zwei vorzugsweise fest oder unbeweglich mit dem Instrumentenhalter verbundenen Markern ausführen kann, wenn das Instrument relativ zu dem Instrumentenhalter verschoben wird.

Das Instrument weist mindestens zwei Funktionselemente auf, so dass das Instrument die selbe Funktionalität wie mindestens zwei Instrumente mit je einer Funktionseinheit aufweist, wobei die Funktionselemente des Instruments bevorzugt unterschiedlich ausgestaltet sind. Des Weiteren wird das Instrument von dem Instrumentenhalter gehalten und kann relativ zu dem Instrumentenhalter und/oder relativ zu den mindestens zwei mit dem Instrumentenhalter vorzugsweise unbeweglich verbundenen Markern bewegt werden. Der Instrumentenhalter ist vorzugsweise hohlzylinderförmig ausgestaltet und enthält in seinem Inneren das Instrument, wobei das Instrument bevorzugt innerhalb des Instrumentenhalters verschiebbar gelagert sein kann und vorzugsweise auch entlang der Längsachse des präkalibrierten Mehrweginstrumentes oder des Instrumentenhalters, zum Beispiel entlang der Längsachse des hohlzylinderförmigen Instrumentenhalters, verschiebbar sein kann. Insbesondere kann der Instrumentenhalter auch als Griff des präkalibrierten Mehrweginstrumentes dienen.

Das Instrument kann zum Beispiel zusammen mit dem mindestens einen mit dem Instrument verbundenen Marker relativ zu dem Instrumentenhalter und/oder relativ zu den mit dem Instrumentenhalter verbundenen Markern bewegt oder verschoben werden, wobei das Instrument vorzugsweise verdrehsicher in dem Instrumentenhalter gelagert ist, um eine möglichst gleichbleibende Bewegung des Instruments relativ zu dem Instrumentenhalter, insbesondere eine möglichst gleichbleibende und/oder spiellose Translationsbewegung, zu gewährleisten.

Das Instrument, das vorzugsweise in dem Instrumentenhalter liegt, kann relativ zu dem Instrumentenhalter in zwei feste Endzustände bewegt werden, in denen das Instrument zum Beispiel einrastet und zunächst nicht mehr weiter bewegt oder verschoben werden kann bis der Endzustand zum Beispiel durch Lösen einer Verrastung gelöst wird und das Instrument wieder bewegt werden kann. Insbesondere können die Endzustände des Instrumentes gesichert sein, so dass das Instrument, solange die Sicherung besteht, nicht bewegt werden kann und erst nach dem Lösen der Sicherung relativ zu dem Instrumentenhalter verschoben werden kann. Die mindestens zwei Funktionselemente des Instruments können identisch oder ähnlich oder verschieden ausgestaltet sein, und können die selbe oder eine ähnliche oder eine verschiedene Funktionalität besitzen und können insbesondere präkalibriert sein, wodurch bekannt sein kann, welches Funktionselement welche Funktionalität ausführen kann oder welche Funktionselemente sich an dem Instrument befinden oder welches Funktionselement bei welcher Markeranordnung oder -geometrie eingesetzt werden kann oder soll. Dabei kann zum Beispiel ein Navigationssystem die jeweilige Markergeometrie erfassen oder erkennen und aus der Geometrie der Markeranordnung, wobei mindestens zwei Marker mit dem Instrumentenhalter und mindestens ein Marker mit dem Instrument verbunden sein kann, darauf schließen, welches Funktionselement verwendet werden soll oder welches Funktionselement benutzt werden kann oder zur Benutzung verfügbar ist. Sind zum Beispiel zwei Marker, bei denen es sich sowohl um aktive als auch um passive Marker handeln kann, mit dem Instrumentenhalter verbunden und ist ein Marker mit dem Instrument verbunden und weist das Instrument zwei Funktionselemente auf, so könnten zum Beispiel in einem Zustand des präkalibrierten Mehrweginstruments, zum Beispiel in einem festen Endzustand, die Marker eine bestimmte Markergeometrie ausbilden, die anzeigt welches der beiden Funktionselemente verwendet werden soll oder zur Benutzung bereit steht. In einem zweiten Zustand des präkalibrierten Mehrweginstruments könnten die Marker eine andere bestimmte Geometrie ausbilden, aus der geschlossen werden kann, dass das zweite Funktionselement verwendet werden kann oder verwendet werden soll.

Bei dem erfindungsgemäßen präkalibrierten Mehrweginstrument kann ein Registrierungsvorgang, bei dem das Mehrweginstrument zum Beispiel von einer Kamera, wie einer Infrarot-Kamera, erfasst wird und dessen Position im Raum ermittelt wird, ausreichen, um die mindestens zwei Funktionselemente, die zum Beispiel unterschiedliche Form haben können und zum Beispiel für die Registrierung unterschiedlicher Körperteile, wie unterschiedlicher Knochen oder Knochenformen, eingesetzt werden können, als Registrierungsinstrumente einsetzen zu können. Damit kann ein Registrierungsgerät, wie das präkalibrierte Mehrweginstrument, ausreichen, um verschiedene Aufgaben auszuführen, während ansonsten mehrere Instrumente, wie Registrierungsgeräte oder Pointer, verwendet werden müssten, die alle einzeln registriert werden müssten, um zum Beispiel für die Registrierung von Knochen oder Knochenkonturen eingesetzt werden zu können. Dahingegen wird das präkalibrierte Mehrweginstrument zum Beispiel einmal registriert und kann bevorzugt von einem Navigationssystem navigiert werden, wobei vorzugsweise von dem Navigationssystem und/oder einer Recheneinheit aufgrund der Markergeometrie erkannt werden kann, welches Funktionselement des präkalibrierten Mehrweginstrumentes eingesetzt werden soll, wobei das zum Einsatz vorgesehene Funktionselement, aufgrund der Präkalibrierung aller Funktionselemente, zum Beispiel für die Registrierung von Knochen oder Knochenkonturen eingesetzt werden kann. Auch kann zum Beispiel ein Kalibriervorgang ausreichen, um das Mehrweginstrument mit den mindestens zwei Funktionselementen zu kalibrieren, während bei der Verwendung von mehreren Instrumenten mit je einem Funktionselement jedes Instument kalibriert werden muss.

Insbesondere können die mindestens zwei Funktionselemente des Instruments an den Enden oder Spitzen des Instrumentes ausgebildet sein, insbesondere als identische oder unterschiedliche Spitzen des vozugsweise länglichen oder stabförmigen Instrumentes, das relativ zu dem Instrumentenhalter zum Beispiel in mindestens zwei feste oder unveränderliche Endzustände bewegt werden kann. In den mindestens zwei festen Endzuständen des Instruments kann vorzugsweise mindestens eines der mindestens zwei Funktionselemente, wie zum Beispiel die Spitzen des Instrumentes, durch den Instrumentenhalter geschützt werden, indem zum Beispiel der Instrumentenhalter das Funktionselement oder die Spitze umgibt, indem sich die geschützte Spitze oder das geschützte Funktionselement zum Beispiel im Inneren des Instrumentenhalters befindet, während das oder die nicht geschützten Funktionselemente zum Beispiel für Registrierungsvorgänge eingesetzt werden können. Besonders bevorzugt ist in jedem Endzustand nur eines der Funktionselemente oder nur eine der Spitzen einsetzbar, während die übrigen Funktionselemente von dem Instrumentenhalter geschützt werden und nicht eingesetzt werden können.

Mindestens einer der Marker kann mit dem Instrument verbunden sein oder kann vorzugsweise direkt auf dem Instrument angeordnet sein und mindestens zwei Marker können mit dem Instrumentenhalter verbunden sein oder auf dem Instrumentenhalter angeordnet sein, so dass mindestens ein auf dem Instrument angeordneter Marker zum Beispiel entlang der Längsachse des präkalibrierten Mehrweginstrumentes oder des Instrumentenhalters verschoben werden kann, insbesondere zusammen mit dem Instrument verschoben werden kann, und somit seine Position relativ zu den mindestens zwei auf dem Instrumentenhalter angeordneten Markern verändert. Aus der neuen Position des mindestens einen auf dem Instrument angeordneten Markers relativ zu den mindestens zwei auf dem Instrumentenhalter vorzugsweise fest oder unbeweglich angeordneten Markern kann geschlossen werden, welches Funktionselement oder welche Funktionselemente benutzt werden können, also zum Beispiel nicht durch den Instrumentenhalter geschützt werden, oder welche Funktionselemente nicht benutzt werden können, weil sie zum Beispiel von dem Instrumentenhalter umgeben werden oder andersartig durch den Instrumentenhalter geschützt werden und nicht eingesetzt werden können.

In einem ersten Endzustand des Instrumentes, in welchem das Instrument vorzugsweise nicht mehr relativ zu dem Instrumentenhalter bewegt werden kann, können die mindestens drei Marker ein Dreieck oder ein Vieleck ausbilden, wobei das Dreieck nicht kongruent zu dem Dreieck ist, welches die mindestens drei Marker in einem weiteren oder in einem zweiten Endzustand des Instrumentes bilden. Aufgrund der Unterschiedlichkeit der Dreiecke oder der Vielecke oder der Geometrie der Markeranordnung in den Endzuständen kann zum Beispiel ein Navigationssystem ermitteln, in welchem Zustand sich das Mehrweginstrument befindet und daraus schließen beziehungsweise ermitteln welches Funktionselement des Instrumentes einsetzbar ist.

Bei dem erfindungsgemäßen Verfahren zum Erkennen des Zustandes des präkalibrierten Mehrweginstrumentes kann ein Zustand von mindestens zwei möglichen Zuständen oder eine Markergeometrie von mindestens zwei möglichen Markergeometrien des Mehrweginstrumentes erkannt werden und der erkannte oder aktuelle Zustand oder die erkannte oder aktuelle Markergeometrie mittels gespeicherter Informationen über die Geometrie des präkalibrierten Mehrweginstrumentes ermittelt werden, insbesondere dadurch, dass der aktuelle Zustand oder die aktuelle Markergeometrie mit einer Vielzahl von bekannten oder gespeicherten Markergeometrien, die zum Beispiel in einer Datenbank oder einem Speicher gespeichert sein können, verglichen wird, woraus geschlossen werden kann, welches der Funktionselemente eingesetzt werden kann.

Die Erfindung bezieht sich weiterhin auf ein Computerprogramm, welches, wenn es in einen Computer geladen wird oder auf einem Computer läuft, ein wie oben beschriebenes Verfahren durchführt. Des Weiteren bezieht sich die Erfindung auf ein Programmspeichermedium oder ein Computerprogrammprodukt mit einem solchen Programm.

Die Erfindung wird nachfolgend anhand bevorzugter Ausführungsbeispiele beschrieben werden. Es zeigen:
- Figur 1: ein erfindungsgemäßes präkalibriertes Mehrweginstrument in einem ersten Zustand;
- Figur 2: das erfindungsgemäße präkalibrierte Mehrweginstrument aus Figur 1 in einem zweiten Zustand.

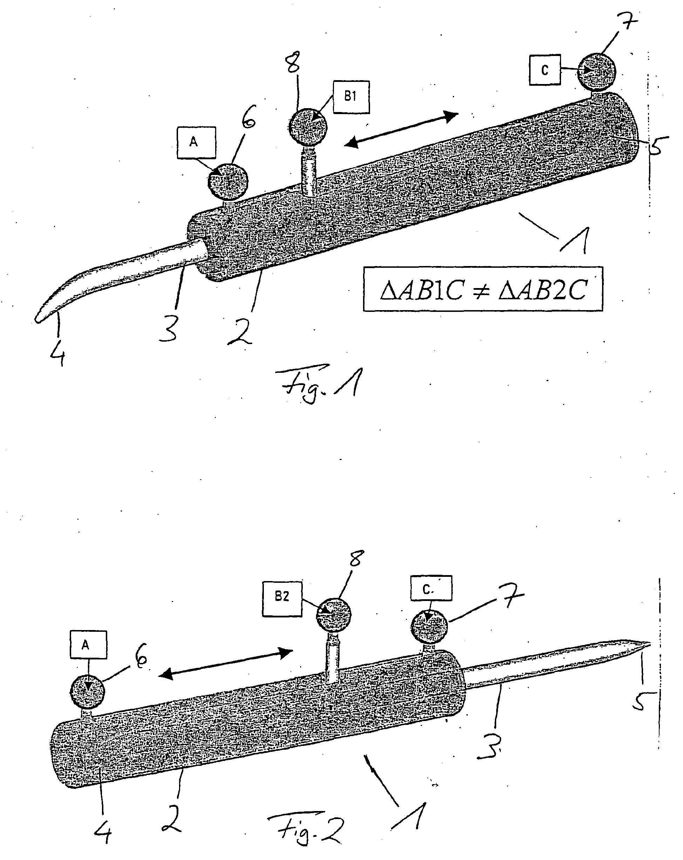

Figur 1 zeigt ein präkalibriertes Mehrweginstrument 1 mit einem hohlzylinderförmigen Instrumentenhalter 2 und einem darin liegenden stabförmigen Instrument 3, welches an seinen Enden unterschiedlich geformte Spitzen 4, 5 aufweist, die eine unterschiedliche Funktionalität besitzen und zum Beispiel vor oder während oder nach einem chirurgischen Eingriff zur Registrierung unterschiedlicher Körperstellen oder Knochenkonturen eingesetzt werden können. Bei dem Instrument 3 kann es sich um einen Pointer-Instrument handeln, mit dessen Spitzen 4, 5 Körper, wie Teile des Körpers eines Patienten oder Knochen, abgefahren oder abgetastet und registriert werden können. Aufgrund der unterschiedlichen Form der Spitzen 4, 5 des Pointer-Instruments können die Spitzen 4, 5 für unterschiedliche Aufgaben, wie die Registrierung unterschiedlicher Konturen oder Knochen oder Körperteile, eingesetzt werden, so dass dieser Zwei-Spitzen-Pointer die selbe Funktionalität besitzt wie zwei Pointer, mit je einer entsprechenden Spitze. An dem Instrumentenhalter 2 sind zwei passive oder aktive Marker 6, 7 an den Positionen A und C angebracht, während an dem Instrument 3 ein Marker 8 befestigt ist. In der Position oder in dem Endzustand B1 des Markers 8 bilden die drei Marker 6, 7, 8 ein Dreieck AB1 C aus, wenn man sich die Marker 6, 7, 8 verbunden denkt. In dem festen Endzustand B1 des Markers 8 ist der Marker 8 vorzugsweise eingerastet oder verrastet oder gesichert, so dass er erst nach Lösen dieser Verrastung oder Sicherung wieder bewegt werden kann, wobei, wie zu erkennen, die eine Spitze 5 des Instrumentes 3 vollständig in dem Instrumentenhalter 2 liegt oder von diesem umgeben ist und so zum Beispiel vor Berührungen oder Beschädigungen oder Brechen geschützt ist, während die andere Spitze 4 für einen Registrierungsvorgang verwendet werden kann. Welche Spitze 4, 5 verwendet werden kann ist eindeutig durch die Geometrie der Markeranordnung, insbesondere durch das durch die Marker 6, 7, 8 gebildete Dreieck AB1 C insofern definiert, dass die Markeranordnung zum Beispiel von einem Navigationssystem erfasst werden kann und mit bekannten Markeranordnungen, wie Dreiecksformen, verglichen werden kann. Dieser Vergleich kann zum Beispiel in einer Recheneinheit, wie einem Computer, durchgeführt werden und aus dem Vergleich des aktuellen Zustands oder der aktuellen Markergeometrie mit bekannten Zuständen oder Markergeometrien kann ermittelt werden, welche Spitze 4, 5 des Pointers oder Instrumentes 3 zum Beispiel zur Registrierung verwendet werden soll, um so spezifische Daten der einsetzbaren Spitze 4 bei der Registrierung eines Körpers oder eines Körperteils, wie einem Knochen, berücksichtigen zu können.

Figur 2 zeigt das präkalibrierte Mehrweginstrument aus Figur 1 in einem zweiten vorzugsweise festen oder gesicherten Endzustand B2 des Markers 8, wobei die Markeranordnung aus Figur 2 vorzugsweise aus jeder Richtung des Raumes von der Markeranordnung aus Figur 1 unterschieden werden kann. Das durch die drei Marker 6, 7, 8 gebildete Dreieck AB2C aus Figur 2 ist nicht kongruent zu dem Dreieck AB1C der Markeranordnung aus Figur 1, da bei einer Betrachtung von jeder Stelle des Raumes entweder unterschiedliche Seiten der beiden Dreiecke AB1C und AB2C festzustellen sind und/oder unterschiedliche Winkel der beiden Dreiecke AB1 C und AB2C zu erkennen sind. Damit kann von jedem Punkt des Raumes klar unterschieden werden, in welcher Position, insbesondere in welchem festen Endzustand, sich der Marker 8 befindet, um daraus zu folgern, welche der beiden Spitzen 4, 5 des Pointers oder des Instruments 3 aus dem Instrumentenhalter 2 herausgefahren ist oder welche der beiden Spitzen 4, 5 in dem Instrumentenhalter 2 liegt. Daraus kann geschlossen werden, welche Daten aufgrund der unterschiedlichen Form der Spitzen 4, 5 bei dem Registrierungsvorgang, insbesondere bei den Berechnungen der Recheneinheit, berücksichtigt werden müssen.

## Patentansprüche

1. Präkalibriertes Mehrweginstrument (1) mit:
- einem Instrumentenhalter (2),
- einem Instrument (3), das mindestens zwei Funktionselemente (4, 5) aufweist und von dem Instrumentenhalter (2) gehalten wird und relativ zu dem Instrumentenhalter (2) bewegt werden kann, wobei die mindestens zwei Funktionselemente (4, 5) an den Enden oder Spitzen des Instrumentes (3) ausgebildet sind; und
- mindestens drei Markern (6, 7, 8), wobei mindestens einer der Marker (8), zusammen mit dem Instrument (3) relativ zu dem Instrumentenhalter (2) bewegt werden kann, wobei mindestens einer der Marker (6, 7, 8) an dem Instrument (3) angeordnet ist;
- wobei der Instrumentenhalter (2) der Griff des präkalibrierten Mehrweginstrumentes (1) ist.

2. Präkalibriertes Mehrweginstrument (1) nach Anspruch 1, wobei das Instrument (3) innerhalb des Instrumentenhalters (2) verschiebbar gelagert ist.

3. Präkalibriertes Mehrweginstrument (1) nach einem der vorhergehenden Ansprüche, wobei das Instrument (3) entlang der Längsachse des präkalibrierten Mehrweginstrumentes (1) oder des Instrumentenhalters (2) verschiebbar ist.

4. Präkalibriertes Mehrweginstrument (1) nach einem der vorhergehenden Ansprüche, wobei das Instrument (3) verdrehsicher in dem Instrumentenhalter (2) gelagert ist.

5. Präkalibriertes Mehrweginstrument (1) nach einem der vorhergehenden Ansprüche, wobei das Instrument (3) in mindestens zwei feste Endzustände (B1, B2) bewegt werden kann.

6. Präkalibriertes Mehrweginstrument (1) nach einem der vorhergehenden Ansprüche, wobei mindestens zwei Funktionselemente (4, 5) des Instruments (3) voneinander verschieden sind.

7. Präkalibriertes Mehrweginstrument (1) nach einem der vorhergehenden Ansprüche, wobei in den mindestens zwei festen Endzuständen (B1, B2) des Instrumentes (3) mindestens eines der mindestens zwei Funktionselemente durch den Instrumentenhalter (2) geschützt wird.

8. Präkalibriertes Mehrweginstrument (1) nach einem der vorhergehenden Ansprüche, wobei mindestens zwei der Marker (6, 7, 8) an dem Instrumentenhalter (2) angeordnet sind.

9. Präkalibriertes Mehrweginstrument (1) nach einem der vorhergehenden Ansprüche, wobei mindestens einer der Marker (6, 7, 8) entlang der Längsachse des präkalibrierten Mehrweginstrumentes (1) oder des Instrumentenhalters (2) verschiebbar ist.

10. Präkalibriertes Mehrweginstrument (1) nach einem der vorhergehenden Ansprüche, wobei in einem erstem Endzustand (B1) des Instrumentes (3) die mindestens drei Marker (6, 7, 8) ein Dreieck AB1C bilden, welches nicht kongruent zu dem Dreieck AB2C ist, welches die mindestens drei Marker (6, 7, 8) in einem zweiten Endzustand (B2) des Instrumentes bilden.

11. Verfahren zum Erkennen des Zustandes eines präkalibrierten Mehrweginstrumentes (1) nach einem der vorhergehenden Ansprüche, wobei eine von mindestens zwei unterschiedlichen möglichen Markergeometrien des präkalibrierten Mehrweginstrumentes (1) erkannt wird und der aktuelle Zustand des präkalibrierten Mehrweginstrumentes (1) mittels gespeicherter Informationen über die Geometrie des präkalibrierten Mehrweginstrumentes (1) ermittelt wird.

12. Computerprogramm, welches, wenn es auf einem Computer läuft oder in einen Computer geladen ist, das Verfahren nach dem vorhergehenden Anspruch durchführt.

13. Programmspeichermedium oder Computerprogrammprodukt mit dem Computerprogramm nach dem vorhergehenden Anspruch.

## Claims

1. A pre-calibrated reusable instrument (1), comprising:
- an instrument holder (2);
- an instrument (3) which comprises at least two functional elements (4, 5) and is held by the instrument holder (2) and can be moved relative to the instrument holder (2), wherein the at least two functional elements (4, 5) are formed on the ends or tips of the instrument (3); and
- at least three markers (6, 7, 8), wherein at least one of the markers (8) can be moved together with the instrument (3), relative to the instrument holder (2), wherein at least one of the markers (6, 7, 8) is arranged on the instrument (3);
- wherein the instrument holder (2) is the grip for the pre-calibrated reusable instrument (1).

2. The pre-calibrated reusable instrument (1) according to claim 1, wherein the instrument (3) is mounted within the instrument holder (2) such that it can be shifted.

3. The pre-calibrated reusable instrument (1) according to any one of the preceding claims, wherein the instrument (3) can be shifted along the longitudinal axis of the pre-calibrated reusable instrument (1) or the instrument holder (2).

4. The pre-calibrated reusable instrument (1) according to any one of the preceding claims, wherein the instrument (3) is mounted non-rotatably in the instrument holder (2).

5. The pre-calibrated reusable instrument (1) according to any one of the preceding claims, wherein the instrument (3) can be moved into at least two fixed end states (B1, B2).

6. The pre-calibrated reusable instrument (1) according to any one of the preceding claims, wherein at least two functional elements (4, 5) of the instrument (3) are different from each other.

7. The pre-calibrated reusable instrument (1) according to any one of the preceding claims, wherein in the at least two fixed end states (B1, B2) of the instrument (3), at least one of the at least two functional elements is protected by the instrument holder (2).

8. The pre-calibrated reusable instrument (1) according to any one of the preceding claims, wherein at least two of the markers (6, 7, 8) are arranged on the instrument holder (2).

9. The pre-calibrated reusable instrument (1) according to any one of the preceding claims, wherein at least one of the markers (6, 7, 8) can be shifted along the longitudinal axis of the pre-calibrated reusable instrument (1) or the instrument holder (2).

10. The pre-calibrated reusable instrument (1) according to any one of the preceding claims, wherein in a first end state (B1) of the instrument (3), the at least three markers (6, 7, 8) form a triangle AB1C which is not congruent with the triangle AB2C which the at least three markers (6, 7, 8) form in a second end state (B2) of the instrument.

11. A method for identifying the state of a pre-calibrated reusable instrument (1) according to any one of the preceding claims, wherein one of at least two different possible marker geometries of the pre-calibrated reusable instrument (1) is identified, and the current state of the pre-calibrated reusable instrument (1) is ascertained by means of stored information on the geometry of the pre-calibrated reusable instrument (1).

12. A computer program which, when it is running on a computer or is loaded onto a computer, performs the method according to the preceding claim.

13. A program storage medium or computer program product comprising the computer program according to the preceding claim.

## Revendications

1. Instrument multi-usages précalibré (1) comportant :
- un porte-instruments (2),
- un instrument (3), qui présente au moins deux éléments fonctionnels (4, 5) et est maintenu par le porte-instruments (2) et peut être déplacé par rapport au porte-instruments (2), les au moins deux éléments fonctionnels (4, 5) étant réalisés au niveau des extrémités ou des pointes de l'instrument (3), et
- au moins trois marqueurs (6, 7, 8), au moins un des marqueurs (8) pouvant être déplacé par rapport au porte-instruments (2) en commun avec l'instrument (3), au moins un des marqueurs (6, 7, 8) étant agencé au niveau de l'instrument (3),
- le porte-instruments (2) étant la poignée de l'instrument multi-usages précalibré (1) .

2. Instrument multi-usages précalibré (1) selon la revendication 1, dans lequel l'instrument (3) est logé à l'intérieur du porte-instruments (2) de manière à pouvoir être déplacé.

3. Instrument multi-usages précalibré (1) selon l'une quelconque des revendications précédentes, dans lequel l'instrument (3) peut être déplacé le long de l'axe longitudinal de l'instrument multi-usages précalibré (1) ou du porte-instruments (2).

4. Instrument multi-usages précalibré (1) selon l'une quelconque des revendications précédentes, dans lequel l'instrument (3) est logé de manière bloquée en rotation dans le porte-instruments (2).

5. Instrument multi-usages précalibré (1) selon l'une quelconque des revendications précédentes, dans lequel l'instrument (3) peut être amené dans au moins deux états finaux (B1, B2) fixes.

6. Instrument multi-usages précalibré (1) selon l'une quelconque des revendications précédentes, dans lequel au moins deux éléments fonctionnels (4, 5) de l'instrument (3) sont différents les uns des autres.

7. Instrument multi-usages précalibré (1) selon l'une quelconque des revendications précédentes, dans lequel au moins un des au moins deux éléments fonctionnels est protégé par le porte-instruments (2), dans les au moins deux états finaux (B1, B2) fixes de l'instrument (3).

8. Instrument multi-usages précalibré (1) selon l'une quelconque des revendications précédentes, dans lequel au moins deux des marqueurs (6, 7, 8) sont agencés au niveau du porte-instruments (2).

9. Instrument multi-usages précalibré (1) selon l'une quelconque des revendications précédentes, dans lequel au moins un des marqueurs (6, 7, 8) peut être déplacé le long de l'axe longitudinal de l'instrument multi-usages précalibré (1) ou du porte-instruments (2).

10. Instrument multi-usages précalibré (1) selon l'une quelconque des revendications précédentes, dans lequel les au moins trois marqueurs (6, 7, 8) forment, dans un premier état final (B1) de l'instrument (3), un triangle AB1C qui n'est pas congruent au triangle AB2C que forment les au moins trois marqueurs (6, 7, 8) dans un second état final (B2) de l'instrument (3).

11. Procédé d'identification de l'état d'un instrument multi-usages précalibré (1) selon l'une quelconque des revendications précédentes, dans lequel une géométrie de marqueurs, parmi au moins deux géométries de marqueurs différentes possibles, de l'instrument multi-usages précalibré (1) est identifiée et l'état actuel de l'instrument multi-usages précalibré (1) est déterminé grâce à des informations stockées concernant la géométrie de l'instrument multi-usages précalibré (1).

12. Logiciel informatique, qui, lorsqu'il fonctionne dans un ordinateur ou est chargé dans un ordinateur, met en oeuvre le procédé selon la revendication précédente.

13. Support de stockage de logiciel ou produit logiciel informatique comprenant le logiciel informatique selon la revendication précédente.
